# EUROPEAN PATENT APPLICATION

(11) **EP 2 359 806 A1**
(43) Date of publication of application: **24.08.2011**
(21) Application number: 10195612.6
(22) Date of filing: 17.12.2010
(51) Int. Cl.: A61K 8/87, A61K 8/04, A61K 8/84, A61Q 1/10, A61Q 1/06, A61K 8/92

(54) **Composition containing an aqueous dispersion of polyurethane and an oil-soluble polar modified polymer**

(30) Priority: 18.12.2009 US 287945 P; 18.12.2009 US 288022 P
(71) Applicant: L'OREAL S.A., 75008 Paris Cedex (FR)
(72) Inventor: Bui, Hy Si, Piscataway, NJ NJ 08854 (US); Kanji, Mohamed, Edison, NJ NJ 08837 (US); Tong, Anita Chon, Westfield, NJ NJ 07090 (US); Li, Chunhua, North Plainfield, NJ NJ 07060 (US); Bavouzet, Bruno Thierry, 94250, Gentilly (FR)
(74) Representative: Le Coupanec, Pascale A.M.P.

(57) **Abstract**

The invention relates to a composition comprising at least one aqueous polyurethane dispersion, at least one oil-soluble polar modified polymer, and at least one polyamine compound having at least two amine groups.

## Description

### CROSS-REFERENCE TO RELATED APPLICATIONS

This application claims the benefit of priority under 35 U.S.C. § 119(e) from U.S. Provisional Application Serial Nos. 61/287,945 and 61/288,022, both filed December 18, 2009.

### FIELD OF THE INVENTION

The present invention relates to compositions comprising at least one aqueous dispersion of polyurethane and at least one oil-soluble polymer modified polymer. The compositions further comprise at least one polyamine compound having at least two amine groups. The compositions have beneficial cosmetic properties including but not limited to good smudge resistance, good volumizing properties, good curling properties, good curl retention properties, the ability to be easily removed with water, improved waterproof characteristics, oil-resistance, improved feel upon application (for example, texture, reduced drag or tackiness), improved shine/color characteristics, and/or increased long wear properties.

### DISCUSSION OF THE BACKGROUND

In the past, long-wear, smudge-resistant mascaras were not washable with water. Such mascaras were typically anhydrous.

In contrast, mascara compositions which were washable with water were not long-wear or smudge-resistant. Such mascaras typically contained significant amounts of water (for example, oil-in-water emulsions).

Given these countervailing considerations, it has been difficult to prepare long-wear, smudge-resistant mascaras which are washable with water.

Thus, there remains a need for improved mascara compositions which have desired wear properties as well as desired removal properties.

Further, lipsticks and foundations are typically used to enhance natural features by adding color and/or shine to the lip or skin area. Generally, lipsticks and other lip compositions as well as foundations contain a particulate material such as pigment or particulate fillers in an oil and/or wax base. See, e.g., U.S. application Publication no. 2004/0161395.

The cosmetic industry has endeavored to provide long lasting cosmetics with good wear properties. However, such compositions remain elusive.

There thus also remains a need for improved lip or skin compositions which have desired wear and feel properties.

### SUMMARY OF THE INVENTION

The present invention relates to compositions comprising at least one aqueous polyurethane dispersion, at least one oil-soluble polar modified polymer, and at least one polyamine compound having at least two amine groups.

The present invention relates to compositions comprising (i) at least one aqueous polyurethane dispersion, and (ii) a reaction product of at least one oil-soluble polar modified polymer and at least one polyamine compound having at least two amine groups.

The present invention relates to compositions made by combining ingredients comprising at least one aqueous polyurethane dispersion, at least one oil-soluble polar modified polymer, and at least one polyamine compound having at least two amine groups.

The present invention relates to lip, eyelash or skin compositions, preferably lipsticks, mascaras or stick foundations, comprising at least one aqueous polyurethane dispersion, at least one oil-soluble polar modified polymer, and at least one polyamine compound having at least two amine groups.

The present invention relates to lips, eyelash or skin compositions, preferably lipsticks, mascaras or stick foundations, comprising (i) at least one aqueous polyurethane dispersion, and (ii) a reaction product of at least one oil-soluble polar modified polymer and at least one polyamine compound having at least two amine groups.

The present invention also relates to the above compositions further comprising a desired agent such as a colorant and/or pharmacologically active agent.

The present invention also relates to methods of improving the smudge-resistance properties of a lip or skin composition upon application to lips or skin, and/or the adhesion, long-wear and/or transfer-resistance properties of a lip or skin composition, comprising adding to a composition at least one aqueous polyurethane dispersion, at least one oil-soluble polar modified polymer, and at least one polyamine compound having at least two amine groups.

The present invention relates to hair colorant or styling compositions as described above.

The present invention relates to mascara compositions as described above.

The present invention also relates to compositions as described above in the form of a stick.

The present invention also relates to methods of treating, caring for and/or making up keratinous material (for example, hair, skin, eyelashes or lips) by applying cosmetic compositions of the present invention to the keratinous material in an amount sufficient to treat, care for and/or make up the keratinous material.

The present invention also relates to methods of improving the smudge-resistance properties of a cosmetic composition upon application to a keratin material, and/or the adhesion, volumizing, long-wear and/or transfer-resistance properties of a cosmetic composition, and/or the curling or curl retention properties of a cosmetic composition, comprising adding to a composition at least one aqueous polyurethane dispersion, at least one oil-soluble polar modified polymer, and at least one polyamine compound having at least two amine groups.

The present invention also relates to methods of making up eyelashes comprising applying a composition of the invention to eyelashes in an amount sufficient to makeup the eyelashes.

The present invention also relates to methods of making up lips or skin comprising applying a composition of the invention to lips or skin in an amount sufficient to makeup the lips or skin.

The present invention also relates to methods of removing mascara from eyelashes comprising removing a mascara composition of the present invention from eyelashes by applying water to the mascara composition in an amount sufficient to remove the composition from the eyelashes.

The present invention also relates to methods of making a composition comprising reacting at least one aqueous polyurethane dispersion, at least one oil-soluble polar modified polymer, and at least one polyamine compound having at least two amine groups to form the composition.

lt is to be understood that both the foregoing general description and the following detailed description are exemplary and explanatory only, and are not restrictive of the invention.

### DETAILED DESCRIPTION OF THE INVENTION

As used herein, the expression "at least one" means one or more and thus includes individual components as well as mixtures/combinations.

Other than in the operating examples, or where otherwise indicated, all numbers expressing quantities of ingredients and/or reaction conditions are to be understood as being modified in all instances by the term "about," meaning within 10% to 15% of the indicated number.

"Film former" or "film forming agent" as used herein means a polymer or resin that leaves a film on the substrate to which it is applied, for example, after a solvent accompanying the film former has evaporated, absorbed into and/or dissipated on the substrate.

"Transfer resistance" as used herein refers to the quality exhibited by compositions that are not readily removed by contact with another material, such as, for example, a glass, an item of clothing or the skin, for example, when eating or drinking. Transfer resistance may be evaluated by any method known in the art for evaluating such. For example, transfer resistance of a composition may be evaluated by a "kiss" test. The "kiss" test may involve application of the composition to human keratin material such as hair, skin or lips followed by rubbing a material, for example, a sheet of paper, against the hair, skin or lips after expiration of a certain amount of time following application, such as 2 minutes after application. Similarly, transfer resistance of a composition may be evaluated by the amount of product transferred from a wearer to any other substrate, such as transfer from the hair, skin or lips of an individual to a collar when putting on clothing after the expiration of a certain amount of time following application of the composition to the hair, . skin or lips. The amount of composition transferred to the substrate (e.g., collar, or paper) may then be evaluated and compared. For example, a composition may be transfer resistant if a majority of the product is left on the wearer's hair, skin or lips. Further, the amount transferred may be compared with that transferred by other compositions, such as commercially available compositions. ln a preferred embodiment of the present invention, little or no composition is transferred to the substrate from the hair, skin or lips.

"Long wear" compositions as used herein, refers to compositions where color remains the same or substantially the same as at the time of application, as viewed by the naked eye, after an extended period of time. Long wear properties may be evaluated by any method known in the art for evaluating such properties. For example, long wear may be evaluated by a test involving the application of a composition to human hair, skin or lips and evaluating the color of the composition after an extended period of time. For example, the color of a composition may be evaluated immediately following application to hair, skin or lips and these characteristics may then be re-evaluated and compared after a certain amount of time. Further, these characteristics may be evaluated with respect to other compositions, such as commercially available compositions.

"Tackiness" as used herein refers to the adhesion between two substances. For example, the more tackiness there is between two substances, the more adhesion there is between the substances. To quantify "tackiness," it is useful to determine the "work of adhesion" as defined by IUPAC associated with the two substances. Generally speaking, the work of adhesion measures the amount of work necessary to separate two substances. Thus, the greater the work of adhesion associated with two substances, the greater the adhesion there is between the substances, meaning the greater the tackiness is between the two substances.

Work of adhesion and, thus, tackiness, can be quantified using acceptable techniques and methods generally used to measure adhesion, and is typically reported in units of force time (for example, gram seconds ("g s")). For example, the TA-XT2 from Stable Micro Systems, Ltd. can be used to determine adhesion following the procedures set forth in the TA-XT2 Application Study (ref: MATl/PO.25), revised January 2000. According to this method, desirable values for work of adhesion for substantially non-tacky substances include less than about 0.5 g s, less than about 0.4 g s, less than about 0.3 g s and less than about 0.2 g s. As known in the art, other similar methods can be used on other similar analytical devices to determine adhesion.

"Waterproof" as used herein refers to the ability to. repel water and permanence with respect to water. Waterproof properties may be evaluated by any method known in the art for evaluating such properties. For example, a mascara composition may be applied to false eyelashes, which may then be placed in water for a certain amount of time, such as, for example, 20 minutes. Upon expiration of the pre-ascertained amount of time, the false eyelashes may be removed from the water and passed over a material, such as, for example, a sheet of paper. The extent of residue left on the material may then be evaluated and compared with other compositions, such as, for example, commercially available compositions. Similarly, for example, a composition may be applied to skin, and the skin may be submerged in water for a certain amount of time. The amount of composition remaining on the skin after the pre-ascertained amount of time may then be evaluated and compared. For example, a composition may be waterproof if a majority of the product is left on the wearer, e.g., eyelashes, skin, etc. ln a preferred embodiment of the present invention, little or no composition is transferred from the wearer.

"Substituted" as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalky groups, oxyalkylene groups, polyoxyalkylene groups, carboxylic acid groups, amine groups, acylamino groups, amide groups, halogen containing groups, ester groups, thiol groups, sulphonate groups, thiosulphate groups, siloxane groups, and polysiloxane groups. The substituent(s) may be further substituted.

"Volatile", as used herein, means having a flash point of less than about 100°C.

"Non-volatile", as used herein, means having a flash point of greater than about 100°C.

The compositions and methods of the present invention can comprise, consist of, or consist essentially of the essential elements and limitations of the invention described herein, as well as any additional or optional ingredients, components, or limitations described herein or otherwise useful.

OIL-SOLUBLE POLAR MODIFIED POLYMER

. According to the present invention, compositions comprising at least one oil-soluble polar modified polymer are provided. "Polar modified polymer" as used herein refers to a hydrophobic homopolymer or copolymer which has been modified with hydrophilic unit(s). "Oil-soluble" as used herein means that the polar modified polymer is soluble in oil.

Suitable monomers for the hydrophobic homopolymers and/or copolymers include, but are not limited to, cyclic, linear or branched, substituted or unsubstituted, C2-C20 compounds such as, for example, styrene, ethylene, propylene, isopropylene, butylene, isobutylene, pentene, isopentene, isoprene, hexene, isohexene, decene, isodecene, and octadecene, including all ranges and subranges therebetween. Preferably, the monomers are C2-C8 compounds, more preferably C2-C6 compounds, and most preferably C2-C4 compounds such as ethylene, propylene and butylene.

Suitable hydrophilic unit(s) include, but are not limited to, maleic anhydride, acrylates, alkyl acrylates such as, for example, methyl acrylate, ethyl acrylate, propyl acrylate, and butyl acrylate, and polyvinylpyrrolidone (PVP).

According to the present invention, the polar modified polymer is oil-soluble: that is, the polymer does not contain a sufficient amount of hydrophilic unit(s) to render the entire polymer water-soluble or oil-insoluble. According to preferred embodiments, the polar modified polymer contains the same amount of hydrophobic monomer as hydrophilic unit (1:1 ratio) or more hydrophobic monomer than hydrophilic unit. According to particularly preferred embodiments, the polar modified polymer contains 50% or less hydrophilic unit(s) (based on weight of the polymer), 40% or less hydrophilic unit(s), 30% or less hydrophilic unit(s), 20% or less hydrophilic unit(s), 10% or less hydrophilic unit(s), 5% or less hydrophilic unit(s), 4% or less hydrophilic unit(s), or 3% or less hydrophilic unit(s).

Preferably, the polar modified polymer has from about 0.5% to about 10% hydrophilic units, more preferably from about 1% to about 8% hydrophilic units by weight with respect to the weight of the polymer, including all ranges and subranges therebetween. Particularly preferred hydrophilically modified polymers are ethylene and/or propylene homopolymers and copolymers which have been modified with maleic anhydride units.

According to preferred embodiments of the present invention, the polar modified polymer is a wax. According to particularly preferred embodiments, the polar modified wax is made via metallocene catalysis, and includes polar groups or units as well as a hydrophobic backbone. Suitable modified waxes include those disclosed in U.S. patent application publication no. 20070031361. Particularly preferred polar modified waxes are C2-C3 polar modified waxes.

In accordance with preferred embodiments of the present invention, the polar modified wax is based upon a homopolymer and/or copolymer wax of hydrophobic monomers and has a weight-average molecular weight Mw of less than or equal to 25 000 g/mol, preferably of 1000 to 22 000 g/mol and particularly preferably of 4000 to 20,000 g/mol, a number-average molecular weight Mn of less than or equal to 15 000 g/mof, preferably of 500 to 12 000 g/mol and particularly preferably of 1000 to 5000 g/mol, a molar mass distribution Mw/Mn in the range from 1.5 to 10, preferably from 1.5 to 5, particularly preferably from 1.5 to 3 and especially preferably from 2 to 2.5, which have been obtained by metallocene catalysis. Also, the polar modified wax preferably has a melting point above 75°C, more preferably above 90°C such as, for example, a melting point between 90°C and 160°C, preferably between 100°C and 150°C, including all ranges and subranges therebetween.

In the case of a copolymer wax, it is preferable to have, based on the total weight of the copolymer backbone, 0.1 to 30% by weight of structural units originating from the one monomer and 70.0 to 99.9% by weight of structural units originating from the other monomer. Such homopolymer and copolymer waxes can be made, for example, by the process described in EP 571 882, using the metallocene catalysts specified therein. Suitable preparation processes include, for example, suspension polymerization, solution polymerization and gas-phase polymerization of olefins in the presence of metallocene catalysts, with polymerization in the monomers also being possible.

Polar modified waxes can be produced in a known manner from the hompopolymers and copolymers described above by oxidation with oxygen-containing gases, for example air, or by graft reaction with polar monomers, for example maleic acid or acrylic acid or derivatives of these acids. The polar modification of metallocene polyolefin waxes by oxidation with air is described, for example, in EP 0 890 583 A1, and the modification by grafting is described, for example, in U.S. Pat. No. 5,998,547.

Acceptable polar modified waxes include, but are not limited to, homopolymers and/or copolymers of ethylene and/or propylene groups which have been modified with hydrophilic units such as, for example, maleic anhydride, acrylate, methacrylate, polyvinylpyrrolidone (PVP), etc. Preferably, the C2-C3 wax has from about 0.5% to about 10% hydrophilic units, more preferably from about 1% to about 8% hydrophilic units by weight with respect to the weight of the wax, including all ranges and subranges therebetween. Particularly preferred hydrophilically modified waxes are ethylene and/or propylene homopolymers and copolymers which have been modified with maleic anhydride units.

Particularly preferred C2-C3 polar modified waxes for use in the present invention are polypropylene and/or polyethylene-maleic anhydride modified waxes ("PEMA," "PPMA." "PEPPMA") commercially available from Clariant under the trade name LICOCARE or LICOCENE, Specific examples of such waxes include products marketed by Clariant under the LicoCare name having designations such as PP207.

Other suitable polar modified polymers include, but are not limited to A-C 573 A (ETHYLENE-MALEIC ANHYDRIDE COPOLYMER; Drop Point, Mettler : 106°C) from Honeywell, A-C 596 A (PROPYLENE-MALEIC ANHYDRIDE COPOLYMER; Drop Point, Mettler 143°C) from Honeywell, A-C 597 (PROPYLENE-MALEIC ANHYDRIDE COPOLYMER; Drop Point, Mettler : 141°C) from Honeywell, ZeMac® copolymers (from VERTELLUS) which are 1:1 copolymers of ethylene and maleic anhydride, polyisobutylene-maleic anhydride sold under the trade name ISOBAM (from Kuraray), polyisoprene-graft-maleic anhydride sold by Sigma Aidrich, poly(maleic anhydride-octadecene) sold by Chevron Philips Chemcial Co., poly (ethylene-co-butyl acrylate-co-mafeic anhydride) sold under the trade name of Lotader (e.g. 2210, 3210, 4210, and 3410 grades) by Arkema, copolymers in which the butyl acrylate is replaced by other alkyl acrylates (including methyl acrylate [grades 3430, 4404, and 4503] and ethyl acrylate [grades 6200, 8200, 3300, TX 8030, 7500, 5500, 4700, and 4720) also sold by Arkema under the Lotader name, and isobutylene maleic anhydride copolymer sold under the name ACO-5013 by lSP.

According to other embodiments of the present invention, the polar modified polymer is not a wax. In accordance with these embodiments of the present invention, the polar modified polymer is based upon a homopolymer and/or copolymer of hydrophobic monomer(s) and has a weight-average molecular weight Mw of less than or equal to 1,000,000 g/mol, preferably of 1000 to 250,000 g/mol and particularly preferably of 5,000 to 50,000 g/mol, including all ranges and subranges therebetween.

In accordance with these embodiments, the polar modified polymer can be of any form typically associated with polymers such as, for example, block copolymer, a grafted copolymer or an alternating copolymer. For example, the polar modified polymer can contain a hydrophobic backbone (such as polypropylene and/or polyethylene) onto which hydrophilic groups (such as maleic anhydride) have been attached by any means including, for example, grafting. The attached groups can have any orienation (for example, atactic, isotactic or syndiotactic along the backbone).

Preferably, the polar modified polymer(s) represent from abut 1% to about 30% of the total weight of the composition, preferably from about 3% to about 20% of the total weight of the composition, preferably from about 4% to about 15%, and preferably from about 5% to about 10%, including all ranges and subranges therebetween.

AQUEOUS POLYURETHANE DISPERSION

According to the present invention, compositions comprising at least one aqueous polyurethane dispersion are provided. "Aqueous polyurethane dispersion" as used herein means the aqueous polyurethane dispersions disclosed in U.S. patent 7,445,770 and/or U.S. patent 7,452,770.

More specifically, the aqueous polyurethane dispersions of the present invention are preferably the reaction products of:

A) a prepolymer according to the formula:

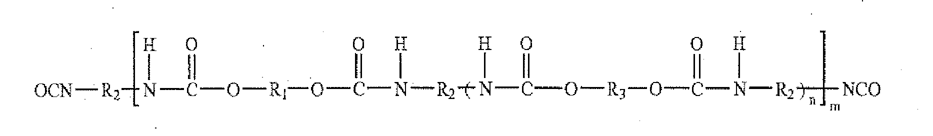

wherein R₁ represents a bivalent radical of a dihydroxyl functional compound, R₂ represents a hydrocarbon radical of an aliphatic or cycloaliphatic polyisocyanate, R₃ represents a radical of a low molecular weight diol, optionally substituted with ionic groups, n is from 0 to 5, and m is >1;

B) at least one chain extender according to the formula: H₂N - R₄—NH₂ wherein R₄ represents an alkylene or alkylene oxide radical not substituted with ionic or potentially ionic groups; and

C) at least one chain extender according to the formula: H₂N —R₅ ―NH₂ wherein R₅ represents an alkylene radical substituted with ionic or potentially ionic groups.

Suitable dihydroxyl compounds for providing the bivalent radical R₁ include those having two hydroxy groups and having number average molecular weights of from about 700 to about 16,000, and preferably from about 750 to about 5000. Examples of the high molecular weight compounds include polyester polyols, polyether polyols, polyhydroxy polycarbonates, polyhydroxy polyacetals, polyhydroxy polyacrylates, polyhydroxy polyester amides, polyhydroxy polyalkadienes and polyhydroxy polythioethers. The polyester polyols, polyether polyols and polyhydroxy polycarbonates are preferred. Mixtures of various such compounds are also within the scope of the present invention.

Suitable polyisocyanates for providing the hydrocarbon radical R₂ include organic diisocyanates having a molecular weight of from about 112 to 1,000, and preferably from about 140 to 400. Preferred diisocyanates are those represented by the general formula R₂(NCO)₂ indicated above in which R₂ represents a divalent aliphatic hydrocarbon group having from 4 to 18 carbon atoms, a divalent cycloaliphatic hydrocarbon group having from 5 to 15 carbon atoms, a divalent araliphatic hydrocarbon group having from 7 to 15 carbon atoms or a divalent aromatic hydrocarbon group having 6-15 carbon atoms. Examples of the organic diisocyanates which are suitable include tetramethylene diisocyanate, 1,6-hexamethylene diisocyanate, dodecamethylene diisocyanate, cyclohexane-1,3- and -1,4-diisocyanate, 1-isocyanato-3-isocyanatomethyl-3,5,5-trimethylcyclohexane (isophorone diisocyanate or lPDl), bis-(4-isocyanatocyclohexyl)-methane, 1,3- and 1,4-bis(isocyanatomethyl)-cyclohexane, bis-(4-isocyanato-3-methyl-cydohexyl)-methane, isomers of toluene diisocyanate (TDl) such as 2,4-diisocyanatotoluene, 2,6-diisocyanatotoluene, mixtures of these isomers, hydrogenated TDI, 4,4'-diisocyanato diphenyl methane and its isomeric mixtures with 2,4'- and optionally 2,2'-diisocyanato diphenylmethane, and 1,5-diisocyanato naphthalene. Mixtures of diisocyanates can, of course, be used. Preferred diisocyanates are aliphatic and cycloaliphatic diisocyanates. Particularly preferred are 1,6-hexamethylene diisocyanate and isophorone diisocyanate.

"Low molecular weight diols" in the context of R₃ means diols having a molecular weight from about 62 to 700, preferably 62 to 200. They may contain aliphatic, alicyclic or aromatic groups. Preferred compounds contain only aliphatic groups. The low molecular weight diols having up to about 20 carbon atoms per molecule include ethylene glycol, diethylene glycol, propane 1,2-diol, propane 1,3-diol, butane 1,4-diol, butylene 1,3-glycol, neopentyl glycol, butyl ethyl propane diol, cyclohexane diol, 1,4-cyclohexane dimethanol, hexane 1,6-diol, bisphenol A (2,2-bis(4-hydroxyphenyl)propane), hydrogenated bisphenol A (2,2-bis(4-hydroxycyclohexyl)propane) , and mixtures thereof. Optionally, the low molecular weight diols may contain ionic or potentially ionic groups. Suitable lower molecular weight diols containing ionic or potentially ionic groups are those disclosed in U.S. Pat. No. 3,412,054. Preferred compounds include dimethylol butanoic acid (DMBA), dimethylol propionic acid (DMBA) and carboxyl-containing caprolactone polyester diol. If lower molecular weight diols containing ionic or potentially ionic groups are used, they are preferably used in an amount such that <0.30 meq of COOH per gram of polyurethane in the polyurethane dispersion are present.

The prepolymer is chain extended using two classes of chain extenders. First, compounds having the formula: H₂N - R₄―NH₂ wherein R₄ represents an alkylene or alkylene oxide radical not substituted with ionic or potentially ionic groups. Alkylene diamines include hydrazine, ethylenediamine, propylenediamine, 1,4-butylenediamine and piperazine. The alkylene oxide diamines include 3-{2-[2-(3-aminopropoxy)ethoxy]ethoxy}propylamine (also known as dipropylamine diethyleneglycol or DPA-DEG available from Tomah Products, Milton, Wis.), 2-methyl-1,5-pentanediamine (Dytec A from DuPont), hexane diamine, isophorone diamine, and 4,4-methylenedi-(cyclohexylamine), and the DPA-series ether amines available from Tomah Products, Milton, Wis., including dipropylamine propyleneglycol, dipropylamine dipropyleneglycol, dipropylamine tripropyleneglycol, dipropylamine poly(propylene glycol), dipropylamine ethyleneglycol, dipropylamine poly(ethylene glycol), dipropylamine 1,3-propane diol, dipropylamine 2-methyl-1,3-propane diol, dipropylamine 1,4-butane diol, dipropylamine 1,3-butane diol, dipropylamine 1,6-hexane diol and dipropylamine cyclohexane-1,4-dimethanol. Mixtures of the listed diamines may also be used.

The second class of chain extenders are compounds having the formula: H₂N ― R₅ -NH₂ wherein R₅ represents an alkylene radical substituted with ionic or potentially ionic groups. Such compounds have an ionic or potentially ionic group and two groups that are reactive with isocyanate groups. Such compounds contain two isocyanate-reactive groups and an ionic group or group capable of forming an ionic group. The ionic group or potentially ionic group can be selected from the group consisting of ternary or quaternary ammonium groups, groups convertible into such a group, a carboxyl group, a carboxylate group, a sulfonic acid group and a sulfonate group. The at least partial conversion of the groups convertible into salt groups of the type mentioned may take place before or during the mixing with water. Specific compounds include diaminosulfonates, such as for example the sodium salt of N-(2-aminoethyl)-2-aminoethane sulfonic acid (AAS) or the sodium salt of N-(2-aminoethyl)-2-aminapropionic acid.

The polyurethane according to the invention may also include compounds which are situated in each case at the chain ends and terminate said chains (chain terminators) as described in U.S. patent 7,445,770 and/or U.S. patent 7,452,770.

Preferably, the aqueous polyurethane dispersion has a viscosity of less than 2000 mPa.s at 23°C, preferably less than 1500, preferably less than 1000, including all ranges and subranges therebetween.

Also preferably, the aqueous polyurethane dispersion has a solids content based on the weigh of the dispersion of from 20% to 60%, preferably from 25% to 55% and preferably from 30% to 50%, including all ranges and subranges therebetween.

According to one embodiment, an aqueous polyurethane suitable for the invention may comprise a hydrophilic portion.

Suitable aqueous polyurethane dispersions for use in the present invention include, but are not limited to, aqueous polyurethane dispersions sold under the BAYCUSANⓇ name by Bayer such as, for example, BAYCUSANⓇ C1000 (polyurethane-34), BAYCUSANⓇ C1001 (polyurethane-34), BAYCUSAN® C1003 (polyurethane-32), and BAYCUSANⓇ C1004 (polyurethane-35).

According to preferred embodiments, the at least one aqueous polyurethane dispersion is present in the composition of the present invention in an amount ranging from about 1 to 40% by weight, preferably from about 2 to about 30% by weight, preferably from about 3 to about 20% by weight, and preferably from about 7 to about 15% by weight based on the total weight of the composition, including all ranges and subranges within these ranges. According to one embodiment, the at least one aqueous polyurethane dispersion is present in the composition of the present invention in an amount ranging from about 1 to 35% by weight based on the total weight of the composition, including all ranges and subranges within these ranges.

POLYAMINE COMPOUND

According to the present invention, compositions comprising at least one polyamine compound are provided. ln accordance with the present invention, the polyamine compound has at least two primary amine groups available to react with hydrophilic groups of the oil-soluble polar modified polymer.

According to particularly preferred embodiments, the polyamine compound is a polyalkyleneimine, preferably a C2-C5 polyalkyleneamine compound, more preferably a polyethyleneimine or polypropyleneimine. Most preferably, the polyalkylenamine is polyethyleneimine ("PEI"). The polyalkyleneamine compound preferably has an average molecular weight range of from 500-200,000, including all ranges and subranges therebetween.

According to preferred embodiments, compositions of the present invention contain polyethyleneimine compounds in the form of branched polymers. Commercially available examples of such polymers are available from BASF under the tradename LUPASOL or POLYIMIN. Non-limiting examples of such polyethyleneimines include Lupasol® PS, Lupasol® PL, Lupasal® PR8515, Lupasol® G20, Lupasol® G35.

According to other embodiments of the present invention, polyamines such as polyethyleneimines and polypropyleneimines can be in the form of dendrimers. Non-limiting examples of such dendrimers are manufactured by the company DSM, and/or are disclosed in U.S. Pat. No. 5,530,092 and U.S. Pat. No. 5,610,268. Commercially available examples of such polymers include polyamidoamine or polypropyleneimine polymers from DENDRITECH sold under the STARBURST® name.

According to other embodiments of the present invention, derivatives of polyalkyleneamines are suitable polyamines. Such derivatives include, but are not limited to, alkylated derivatives, the addition products of alkylcarboxylic acids to polyalkyleneamines, the addition products of ketones and of aldehydes to polyalkyleneamines, the addition products of isocyanates and of isothiocyanates to polyalkyleneamines, the addition products of alkylene oxide or of polyalkylene oxide block polymers to polyalkyleneamines, quaternized derivatives of polyalkyleneamines, the addition products of a silicone to polyalkyleneamines, and copolymers of dicarboxylic acid and polyalkyleneamines, Even further suitable polymamines include, but are not limited to, polyvinylimidazoles (homopolymers or copolymers), polyvinylpyridines (homopolymers or copolymers), compounds comprising vinylimidazole monomers (see, for example, U.S. Pat. No. 5,677,384), and polymers based on amino acids containing a basic side chain (preferably selected from proteins and peptides comprising at least 5%, preferably at least 10% of amino acids selected from histidine, lysine and arginine). Such suitable polyamines as described above include those disclosed and described in U.S. patent 6,162,448. Commercially available examples of such polymers include polyvinylamine/formamide such as those sold under the LupamineⓇ name by BASF, chitosan from vegetable origin such as those sold under the KiosmetineⓇ or Kitozyme® names, or copolymer 845 sold by ISP.

According to preferred embodiments, the at least one polyamine compound is present in the composition of the present invention in an amount ranging from about 0.05 to about 20% by weight, preferably from about 0.25 to about 10% by weight, preferably from about 0.3 to about 5% by weight, preferably from about 0.5 to about 3% by weight, based on the total weight of the composition, including all ranges and subranges within these ranges.

Preferably, the amount of polyamine compound reacted with the oil-soluble polar modified polymer is such that at least two amine groups on the polyamine compound react with the oil-soluble polar modified polymer to form links or bonds between the amine groups and the hydrophilic groups of the oil-soluble polar modified polymer. The appropriate amount of polyamine compound to react with the oil-soluble polar modified polymer to obtain a reaction product can be easily determined, taking into account the number/amount of reactive amine groups on the polyamine compound and the number/amount of corresponding reactive groups on the oil-soluble polar modified polymer (for example, maleic anhydride groups). According to preferred embodiments, excess oil-soluble polar modified polymer (as determined by the relative number/amount of corresponding reactive groups on the polymer as compared to the reactive amine groups on the polyamine) is reacted with polyamine. Preferably, the polyamine to oil-soluble polar modified ratio is between 0.005 and 1, preferably between 0.006 and 0.5, and preferably between 0.007 and 0.1, including all ranges and subranges therebetween.

According to preferred embodiments such as mascara embodiments, the at least one polyamine compound is present in the composition of the present invention in an amount ranging from about 0.25 to about 10% by weight, preferably from about 0.3 to about 5% by weight, preferably from about 0.5 to about 3% by weight, based on the total weight of the composition, including all ranges and subranges within these ranges.

According to preferred embodiments such as lip or skin embodiments, the at least one polyamine compound is present in the composition of the present invention in an amount ranging from about 0.01 to about 5% by weight, preferably from about 0.05 to about 2% by weight, preferably from about 0.1 to about 1% by weight, based on the total weight of the composition, including all ranges and subranges within these ranges.

According to one embodiment, a composition of the invention may be made using from 0.01 to 5% by weight, based on the weight of the composition, of the polyamine.

REACTION PRODUCT

According to one embodiment, the oil-soluble polar modified polymer is reacted with the polyamine compound to form a reaction product

According to preferred embodiments of the present invention, the oil-soluble polar modified polymer is reacted with the polyamine compound, in the presence of water in, at minimum, an amount sufficient to solubilize the polyamine, to form a reaction product. ln accordance with the preferred embodiments, the reaction product is water-insoluble.

Although not wanting to be bound by any particular theory, it is believed that at a temperature below 100°C; the reaction of the oil-soluble polar modified polymer with the primary amine group of the polyamine opens the anhydride ring to form a half acid and half amide crosslinked product. However, at a temperature above 100°C, the reaction of the oil-soluble polar modified polymer with the primary amine group of the polyamine opens the anhydride ring to form an imide crosslinked product. The former product is preferred over the latter product. It is not necessary for all amine groups and all hydrophilic groups to react with each other to form the reaction product. Rather, it is possible that the composition may contain free polyamine and/or free oil-soluble polar modified polymer in addition to the reaction product.

Although not wanting to be bound by any particular theory, it is also believed that the polyamine(s) can be non-covalently assembled with the polar modified polymer(s) by electrostatic interaction between an amine group of the polyamine and a hydrophilic group (for example, carboxylic acid group associated with maleic anhydride groups) of the polar modified polymer to form a supramolecule. For example, with specific reference to maleic anhydride groups, in the presence of water these groups can open to form dicarboxylic acid groups which can interact with protonated primary amines of the polyamine through ionic interaction to form a polymer-polymer complex with hydrophilic core crosslinkers and a hydrophobic network that act as supramolecular capsule. lf a large amount of maleic anhydride groups are present, the secondary amine groups of polyamine are also protonated and interact with alkyl carboxylates.

According to preferred embodiments, the oil-soluble polar modified polymer is in an oil carrier, and the polyamine compound is in an aqueous carrier, and the reaction occurs by combining the oil carrier and the aqueous carrier. Because the oil-soluble polar modified polymer is typically solid at room temperature, the oil carrier is preferably heated to liquefy the polymer prior to combination with the aqueous carrier. Preferably, the oil carrier is heated beyond the melting point of the oil-soluble polar modified polymer, typically up to about 80°C, 90°C or 100°C.

Without intending to be bound by any particular theory, it is believed that the reason for this is that due to the chemical and physical reactions which take place when the oil-soluble polar modified polymer is combined with the polyamine, the subsequent reaction product that is formed is surprisingly and unexpectedly able to entrap large amounts of water molecules within its hydrophobic matrix. The resultant product is eminently capable of forming a film, is self-emulsifying, waterproof. Moreover, the product is both stable and capable of carrying various types of ingredients.

In accordance with the above, compositions of the present invention preferably comprise at least one aqueous polyurethane dispersion, at least one oil-soluble polar modified polymer, and at least one polyamine compound having at least two amine groups.

Compositions of the present invention can optionally further comprise any additive usually used in the field(s) under consideration. For example, dispersants such as poly(12-hydroxystearic acid), antioxidants, oils, sunscreens, preserving agents, fragrances, fillers, neutralizing agents, cosmetic and dermatalogical active agents such as, for example, emollients, moisturizers, vitamins, essential fatty acids, surfactants, silicone elastomers, pasty compounds, viscosity increasing agents such as waxes or liposoluble/lipodispersible polymers, film forming agents, colorants, and mixtures thereof can be added. A non-exhaustive listing of such ingredients can be found in U.S. patent application publication no. 2004/0170586. Further examples of suitable additional components can be found in the other references which have been cited in this application. Still further examples of such additional ingredients may be found in the International Cosmetic Ingredient Dictionary and Handbook (9th ed. 2002).

Suitable oils include volatile and/or non-volatile oils. Such oils can be any acceptable oil including but not limited to silicone oils and/or hydrocarbon oils.

According to certain embodiments, the oil carrier comprises one or more volatile silicone oils. Examples of such volatile silicone oils include linear or cyclic silicone oils having a viscosity at room temperature less than or equal to 6cSt and having from 2 to 7 silicon atoms, these silicones being optionally substituted with alkyl or alkoxy groups of 1 to 10 carbon atoms. Specific oils that may be used in the invention include octamethyltetrasiloxane, decamethyleyclopentasiloxane, dodecamethylcyclahexasifoxane, heptamethyloctyltrisiloxane, hexamethyldisiloxane, decamethyltetrasitoxane, dodecamethylpentasiloxane and their mixtures. Other volatile oils which may be used include KF 96A of 6 cSt viscosity, a commercial product from Shin Etsu having a flash point of 94°C. Preferably, the volatile silicone oils have a flash point of at least 40°C.

Non-limiting examples of volatile silicone oils are listed in Table 1 below.

**Table 1**

| Compound | Flash Point (°C) | Viscosity (cSt) |
|---|---|---|
| Octyltrimethicone | 93 | 1.2 |
| Hexyltrimethicone | 79 | 1.2 |
| Decamethylcyclopentasiloxane | 72 | 4.2 |
| (cyclopentasiloxane or D5) | | |
| Octamethylcyclotetrasiloxane | 55 | 2.5 |
| (cyclotetradimethylsiloxane or D4) | | |
| Dodecamethylcyclohexasiloxane (D6) | 93 | 7 |
| Decamethyltetrasiloxane(L4) | 63 | 1.7 |
| KF-96 A from Shin Etsu | 94 | 6 |
| PDMS (polydimethylsiloxane) DC 200 (1.5cSt) | 56 | 1.5 |
| from Dow Corning | | |
| PDMS DC 200 (2cSt) from Dow Corning | 87 | 2 |

Further, a volatile linear silicone oil may be employed in the present invention. Suitable volatile linear silicone oils include those described in U.S. patent no. 6,338,839 and WO03/042221. ln one embodiment the volatile linear silicone oil is decamethyltetrasiloxane. ln another embodiment, the decamethyltetrasiloxane is further combined with another solvent that is more volatile than decamethyltetrasiloxane.

According to other embodiments, the oil carrier comprises one or more non-silicone volatile oils and may be selected from volatile hydrocarbon oils, volatile esters and volatile ethers. Examples of such volatile non-silicone oils include, but are not limited to, volatile hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, and for example, the oils sold under the trade names of lsopar or Permethyl. Preferably, the volatile non-silicone oils have a flash point of at least 40°C.

Non-limiting examples of volatile non-silicone volatile oils are given in Table 2 below.

**Table 2**

| Compound | Flash Point **(°C)** |
|---|---|
| Isododecane | 43 |
| Propylene glycol n-butyl ether | 60 |
| Ethyl.3-ethoxypropionate | 58 |
| Propylene glycol methylether acetate | 46 |
| lsopar L (isoparaffin C₁₁-C₁₃) | 62 |
| lsopar H (isoparaffin C₁₁-C₁₂ | 56 |

The volatility of the solvents/oils can be determined using the evaporation speed as set forth in U.S. patent no. 6,338,839.

According to other embodiments of the present invention, the oil carrier comprises at least one non-volatile oil. Examples of non-volatile oils that may be used in the present invention include, but are not limited to, polar oils such as:

- hydrocarbon-based plant oils with a high triglyceride content consisting of fatty acid esters of glycerol, the fatty acids of which may have varied chain lengths, these chains possibly being linear or branched, and saturated or unsaturated; these oils are especially wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, for instance those sold by the company Stearineries Dubois or those sold under the names Miglyol 810,812 and 818 by the company Dynamit Nobel;

- synthetic oils or esters of formula R₅COOR₆ in which R₅ represents a linear or branched higher fatty acid residue containing from 1 to 40 carbon atoms, including from 7 to 19 carbon atoms, and R₆ represents a branched hydrocarbon-based chain containing from 1 to 40 carbon atoms, including from 3 to 20 carbon atoms, with R₆ + R₇ ≥ 10, such as, for example, Purcellin oil (cetostearyl octanoate), isononyl isononanoate, C₁₂ to C₁₅ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate; and pentaerythritol esters;

- synthetic ethers containing from 10 to 40 carbon atoms;

- C₈ to C₂₆ fatty alcohols, for instance oleyl alcohol; and

- mixtures thereof.

Further, examples of non-volatile oils that may be used in the present invention include, but are not limited to, non-polar oils such as branched and unbranched hydrocarbons and hydrocarbon waxes including polyolefins, in particular Vaseline (petrolatum), paraffin oil, squalane, squalene, hydrogenated polyisobutene, hydrogenated polydecene, polybutene, mineral oil, pentahydrosqualene, and mixtures thereof.

According to preferred embodiments of the present invention, the compositions can further comprise a desired agent. The desired agent can be, for example, any colorant (pigment, dye, etc.), any pharmaceutically or cosmetically active agent, or any film forming agent known in the art. For example, a cosmetic makeup composition or a paint composition comprising colorant can provide colorant and/or filim forming agent to a substrate (skin, lips, wall, frame, etc.) during use to provide the substrate with the desired film and/or color Similarly, a pharmaceutical or cosmetic composition comprising a pharmaceutically active agent can provide such active agent to the patient or consumer upon use.

Acceptable colorants include pigments, dyes, such as liposoluble dyes, nacreous pigments, and pearling agents.

Representative liposoluble dyes which may be used according to the present invention include Sudan Red, DC Red 17, DC Green 6, ß-carotene, soybean oil, Sudan Brown, DC Yellow 11, DC Violet 2, DC Orange 5, annatto, and quinoline yellow.

Representative nacreous pigments include white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride.

Representative pigments include white, colored, inorganic, organic, polymeric, nonpolymeric, coated and uncoated pigments. Representative examples of mineral pigments include titanium dioxide, optionally surface-treated, zirconium oxide, zinc oxide, cerium oxide, iron oxides, chromium oxides, manganese violet, ultramarine blue, chromium hydrate, and ferric blue. Representative examples of organic pigments include carbon black, pigments of D & C type, and lakes based on cochineal carmine, barium,

Acceptable film forming agents and/or rheological agents are known in the art and include, but are not limited to, those disclosed in U.S. patent application publication no. 2004/0170586.

Non-limiting representative examples of acceptable film forming/rheolgocial agents include silicone resins such as, for example, MQ resins (for example, trimethylsiloxysilicates), T-propyl silsesquioxanes and MK resins (for example, polymethylsilsesquioxanes), silicone esters such as those disclosed in U.S. Pat. Nos. 6,045,782, 5,334,737, and 4,725,658, polymers comprising a backbone chosen from vinyl polymers, methacrylic polymers, and acrylic polymers and at least one chain chosen from pendant siloxane groups and pendant fluorochemical groups such as those disclosed in U.S. Pat. Nos. 5,209,924, 4,693,935, 4,981,903, 4,981,902, and 4,972,037, and WO 01/32737, polymers such as those described in U.S. Pat. No. 5,468,477 (a non-limiting example of such polymers is poly(dimethylsiloxane)-g-poly(isobutyl methacrylate), which is commercially available from 3M Company under the tradename VS 70 IBM).

Suitable examples of acceptable liposoluble polymers include, but are not limited to, polyalkylenes, polyvinylpyrrolidone (PVP) or vinylpyrrolidone (VP) homopolymers or copolymers, copolymers of a C₂ to C₃₀, such as C₃ to C₂₂ alkene, and combinations thereof. As specific examples of VP copolymers which can be used in the invention, mention may be made of VP/vinyl acetate, VP/ethyl methacrylate, butylated polyvinylpyrrolidone (PVP), VP/ethyl methacrylate/methacrylic acid, VP/eicosene, VP/hexadecene, VP/triacontene, VP/styrene or VP/acrylic acid/lauryl methacrylate copolymer.

One type of block copolymer which may be employed in the compositions of the present invention is a thermoplastic elastomer. The hard segments of the thermoplastic elastomer typically comprise vinyl monomers in varying amounts. Examples of suitable vinyl monomers include, but are not limited to, styrene, methacrylate, acrylate, vinyl ester, vinyl ether, vinyl acetate, and the like.

The soft segments of the thermoplastic elastomer typically comprise olefin polymers and/or copolymers which may be saturated, unsaturated, or combinations thereof. Suitable olefin copolymers may include, but are not limited to, ethylene/propylene copolymers, ethylene/butylene copolymers, propylene/butylene copolymers, polybutylene, polyisoprene, polymers of hydrogenated butanes and isoprenes, and mixtures thereof.

Thermoplastic elastomers useful in the present invention include block copolymers e.g., di-block, tri-block, multi-block, radial and star block copolymers, and mixtures and blends thereof. A di-block thermoplastic elastomer is usually defined as an A-B type or a hard segment (A) followed by a soft segment (B) in sequence. A tri-block is usually defined as an A-B-A type copolymer or a ratio of one hard, one soft, and one hard segment. Multi-block or radial block or star block thermoplastic elastomers usually contain any combination of hard and soft segments, provided that the elastomers possess both hard and soft characteristics.

ln preferred embodiments, the thermoplastic elastomer of the present invention may be chosen from the class of Kraton ™ rubbers (Shell Chemical Company) or from similar thermoplastic elastomers. Kraton ™ rubbers are thermoplastic elastomers in which the polymer chains comprise a di-block, tri-block, multi-block or radial or star block configuration or numerous mixtures thereof. The Kraton ™ tri-block rubbers have polystyrene (hard) segments on each end of a rubber (soft) segment, while the Kraton ™ di-block rubbers have a polystyrene (hard) segment attached to a rubber (soft) segment. The Kraton™ radial or star configuration may be a four-point or other multipoint star made of rubber with a polystyrene segment attached to each end of a rubber segment. The configuration of each of the Kraton™ rubbers forms separate polystyrene and rubber domains.

Each molecule of Kraton™ rubber is said to comprise block segments of styrene monomer units and rubber monomer and/or co-monomer units. The most common structure for the Kraton™ triblock copolymer is the linear A-B-A block type styrene-butadiene-styrene, styrene-isoprene-styrene, styrene-ethyfenepropyfene-styrene, or styrene-ethylenebutylene-styrene. The Kraton™ di-block is preferably the AB block type such as styrene-ethylenepropylene, styrene-ethylenebutylene, styrene-butadiene, or styrene-isoprene. The Kraton™ rubber configuration is well known in the art and any block copolymer elastomer with a similar configuration is within the practice of the invention. Other block copolymers are sold under the tradename Septon (which represent elastomers known as SEEPS, sold by Kurary, Co., Ltd) and those sold by Exxon Dow under the tradename Vector™

Other thermoplastic elastomers useful in the present invention include those block copolymer elastomers comprising a styrene-butylene/ethylene-styrene copolymer (tri-block), an ethylene/propylene-styrene copolymer (radial or star block) or a mixture or blend of the two. (Some manufacturers refer to block copolymers as hydrogenated block copolymers, e.g. hydrogenated styrene-butylene/ethylene-styrene copolymer (tri-block)).

Acceptable film forming/rheological agents also include water soluble polymers such as, for example, high molecular weight crosslinked homopolymers of acrylic acid, and Acrylates/C10-30 Alkyl Acrylate Crosspolymer, such as the Carbopol® and Pemulen®; anionic acrylate polymers such as Salcare® AST and cationic acrylate polymers such as Salcare® SC96; acrylamidopropylttrimonium chloride/acrylamide; hydroxyethyl methacrylate polymers, Steareth-10 Allyl Ether/Acrylate Copolymer; Acrylates/Beheneth-25 Metacrylate Copolymer, known as Aculyn® 28; glyceryl polymethacrylate, Acrylates/Steareth-20 Methacrylate Copolymer; bentonite; gums such as alginates, carageenans, gum acacia, gum arabic, gum ghatti, gum karaya, gum tragacanth, guar gum; guar hydroxypropyltrimonium chloride, xanthan gum or gellan gum; cellulose derivatives such as sodium carboxymethyl cellulose, hydroxyethyl cellulose, hydroxymethyl carboxyethyl cellulose, hydroxymethyl carboxypropyl cellulose, ethyl cellulose, sulfated cellulose, hydroxypropyl cellulose, methyl cellulose, hydroxypropylmethyl cellulose, microcrystalline cellulose; agar; pectin; gelatin; starch and its derivatives; chitosan and its derivatives such as hydroxyethyl chitosan; polyvinyl alcohol, PVM/MA copolymer, PVM/MA decadiene crosspolymer, poly(ethylene oxide) based thickeners, sodium carbomer, and mixtures thereof.

According to preferred embodiments of the present invention, the compositions comprise substantial amounts of water. Preferably, compositions of the present invention comprise sufficient water to form a water-in-oil emulsion. Preferably, compositions of the present invention comprise from about 5% to about 80% water, more preferably from about 15% to about 60% water, and more preferably from about 20% to about 50% water by weight with respect to the total weight of the composition, including all ranges and subranges therebetween.

According to one embodiment, a composition of the invention may further comprise water, and preferably in an amount of from 0.1 to 50% by weight, based on the weight of the composition, and more preferably in an amount of from about 5% to about 50% by weight, based on the weight of the composition.

In one embodiment of the present invention, the compositions of the present invention are substantially free of silicone oils (i.e., contain less than about 0.5 % silicone oils).ln another embodiment, the compositions are substantially free of non-silicone oils (i.e., contain less than about 0.5% non-silicone oils). In another embodiment, the compositions are substantially free of non-volatile oils (i.e., contain less than about 0.5% non-volatile oils).

One preferred embodiment of the present invention is an emulsion which is substantially free of surfactant (that is, less than 3% of surfactant), essentially free of surfactant (that is, less than 2% surfactant), or free of surfactant (that is, less than 0.5% surfactant).

Another preferred embodiment of the present invention is a composition which contains so little elastomer that the presence of such elastomer not affect the cosmetic properties of the composition. Preferably, the compositions are substantially free of such elastomers (i.e., contain less than about 0.5% elastomer), essentially free of such elastomers (i.e., contain less than about 0.25%elastomers) or free of such elastomer (i.e., contain no elastomer).

According to other preferred embodiments, methods of treating, caring for and/or enhancing the appearance of keratinous material by applying compositions of the present invention to the keratinous material in an amount sufficient to treat, care for and/or enhance the appearance of the keratinous material are provided. ln accordance with these preceding preferred embodiments, the compositions of the present invention are applied topically to the desired area of the keratin material in an amount sufficient to treat, care for and/or enhance the appearance of the keratinous material. The compositions may be applied to the desired area as needed, preferably once or twice daily, more preferably once daily and then preferably allowed to dry before subjecting to contact such as with clothing or other objects (for example, a glass or a topcoat). Preferably, the composition is allowed to dry for about 1 minute or less, more preferably for about 45 seconds or less. The composition is preferably applied to the desired area that is dry or has been dried prior to application, or to which a basecoat has been previously applied.

According to a preferred embodiment of the present invention, compositions having improved cosmetic properties such as, for example, improved waterproof characteristics, oil-resistance, improved feel upon application (for example, texture, reduced drag or tackiness), increased anti-smudging properties, shine/color characteristics, increased volume properties and/or increased long wear properties are provided.

According to other embodiments of the present invention, methods of improving the anti-smudging, volumizing, waterproof, transfer-resistance and/or long wear properties of a composition, comprising adding at least one polar modified polymer, at least one polyamine, and at least one aqueous polyurethane dispersion are provided.

According to other embodiments, methods of removing mascara from eyelashes comprising removing a mascara composition of the present invention from eyelashes by applying water to the mascara composition in an amount sufficient to remove the composition from the eyelashes are provided.

According to other embodiments of the present invention, methods of improving the anti-smudging, waterproof, transfer-resistance and/or long wear properties of a lip or skin composition, comprising adding at least one polar modified polymer, at least one polyamine, and at least one aqueous polyurethane dispersion to the composition are provided.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective measurements. The following examples are intended to illustrate the invention without limiting the scope as a result. The percentages are given on a weight basis.

**Examples**

**Example 1 - Mascara Composition**

| | **lNCl** | **Inventive Example 1 Amount (weight %)** |
|---|---|---|
| **1** | **Caprylic/capric triglyceride** | **1.00** |
| **2** | **Phenoxyethanol** | **0.50** |
| **3** | **Polyalkylene/maleic** | **9.33** |
| | **anhydride wax in** | |
| | **isohexadecane*** | |
| **4** | **lron oxides** | **8.00** |
| **5** | **lsododecane** | **29.92** |
| **6** | **Ethylparaben** | **0.20** |
| **7** | **Water** | **25.00** |
| **8** | **Disodium EDTA** | **0.10** |
| **9** | **Potassium cetyl phosphate** | **2.00** |
| **10** | **Methyl paraben** | **0.35** |
| **11** | **Pentylene glycol** | **2.00** |
| **12** | **PEI-35** | **1.50** |
| | **(50%solid/50%water)** | |
| **13** | **Simethicone** | **0.10** |
| **14** | **Aqueous polyurethane** | **20.00** |
| | **polyester dispersion** | |
| | **(Baycusan 1004) (41%** | |
| | **solid/60% water)** | |

| | | |
|---|---|---|
| **(*) PP207, which is a linear polypropylene-ethylene-maleic anhydride copolymer wax commercially available from Clariant under the tradename LICOCARE PP207 LP 3349. Material is supplied in 25% isohexadecane.** | | |

**PROCEDURE**

In metal container A, 1, 3, 5 and 6 were added and heated until 90°C (solids melted and became uniform). 4 was added and homogenized at 900 RPM for 1 hour.

ln side beaker B, 10 and 11 were mixed, and heated until 70°C (mixture became clear).

In side tank C with water bath, 7-9 were added, mixed until uniform, and heated until 90°C. Contents of side beaker B were added along with 12.

ln side beaker D, 14 was heated to 90°C, and then added to side tank C.

Side tank C was then mixed for 20 minutes.

When both metal container A and side tank C were at the same temperature 85°C, the contents of side tank C were slowly added to metal container A while homogenizing at 500 RPM.

After the mixture was uniform, 13 was added, and the mixture was mixed mechanically with a stirring rod until uniform.

Then, the mixture began to be cooled naturally. 2 was added to the mixture at 55°C, Then, cooling continued to 25°C.

Example 2 - Comparative Mascara Example

| | **Ingredient** | **Comparative Example 2 Amount (weight %)** |
|---|---|---|
| **1** | **Caprylic/capric triglyceride** | **1.00** |
| **2** | **Polyalkylene/maleic anhydride wax in isohexadecane** | **9.33** |
| **3** | **Iron oxides** | **8.00** |
| **4** | **isododecane** | **40** |
| **5** | **Ethylparaben** | **0.20** |
| **6** | **Water** | **34.42** |
| **7** | **Disodium EDTA** | **0.10** |
| **8** | **Potassium cetyl phosphate** | **2.00** |
| **9** | **Methyl paraben** | **0.35** |
| **10** | **Pentylene glycol** | **2.00** |
| **11** | **PEl-35(50%solid/50%water)** | **2.00** |
| **12** | **Simethicone** | **0.10** |
| **13** | **Phenoxyethanol** | **0.50** |

Similar mascara compositions were prepared. Comparative Example 2 contained a polar modified wax (PP207) and PEl (control). Invention Example 1 contained a polar modified wax (PP 207), PE! and an aqueous polyurethane dispersion (Baycusan 1004).

Comparing with the control (example 2 PPMA+PEl), lt was found that the Example 1 (PPMA+PEl+PU)provides better smudge-resistance and water-resistance. The volume and easy removal properties are also greatly improved. It was also found with increasing the concentration of polyurethane dispersion (PU), the tube removability was able to be achieved.

The Zero Shear viscosity 0.1 1/s for control example 2 was 1797 Pa.sec, whereas it was higher for invention example 1 (4000 Pa.sec).

Examples 3-5 Lipsticks

| Phase | Chemical Name | Comparative example 3 | Inventive example 4 | Inventive example 5 |
|---|---|---|---|---|
| A | Sucrose Acetate isobutyrate | 3.00 | 3.00 | 3.00 |
| | | | | |
| A | **Polyalkylene/maleic anhydride wax in isohexadecane** | 10.00 | 10.00 | 10.00 |
| A | Lauroyl Lysine | 0.30 | 0.30 | 0.30 |
| A | Polyethylene | **11.00** | 11.00 | 11.00 |
| A | Ozokerite | 0.50 | 0.50 | 0.50 |
| A | Color Pigment + Titanium dioxide | 6.75 | 6.75 | 6.75 |
| | Hydrogenated Styrene/Methyl A Styrene/indene Copolymer | 3.27 | 3.27 | 3.27 |
| A | lsododecane | QS | QS | QS |
| B | Deionized Water | 15.00 | 7.50 | 0.00 |
| B | b-Poiyethyleneimine (50% solid/50%water) 0.25 | | 0.25 | 0.25 |
| B | Glycerin | 3.00 | 3.00 | 3.00 |
| B | Baycusan C 1004 40% active: 60% water | 0.00 | 12.50 | 25.00 |

The Zero Shear viscosity 0.1 1/s for control example 2 was 1797 Pa.sec, whereas it was higher for invention example 1 (4000 Pa.sec).

Procedure

All ingredients indicated in phase A were added to a suitable size metal container. The contents were heated to 87 Celsius degrees or until all solids had melted.

All ingredients indicated in phase B were added to a suitable size side tank B, and mixed until uniform. The contents were also heated to 87 Celsius degrees.

When both tanks were at their proper temperatures, side tank B was slowly added to main tank A while homogenizing at 1300 rpm using VMI Turbo Test Rayneri mixer.

Contents were homogenized for 26 minutes.

The contents were poured into lipstick molds at 87 Celsius degrees.

The lipstick in molds was placed in a cooling tunnel for 15 minutes at -10 Celsius degrees. Once cooled, the lipstick in molds were removed from the cooling tunnel to equilibrate to 25 Celsius degrees and removed from mold after lipsticks had thawed to 25 Celsius degrees.

Similar Lipstick compositions were prepared. Comparative Example 3 contained a polar modified wax (PP207) and PEl (control).lnvention Example 4 and invention example 5 contained a polar modified wax (PP 207), PEl and an aqueous polyurethane dispersion (Baycusan 1004).

Comparing with the control (example 3 PPMA+PEl), it was found that the Example 4 (PPMA+PEI+PU) provides better oil resistance with good comfort. The oil resistance was further improved with Example 5 which used PU as the main phase B. It showed further improved in stick structure.

## Claims

1. A composition comprising at least one aqueous polyurethane dispersion, at least one oil-soluble polar modified polymer, and at least one polyamine compound having at least two amine groups.

2. The composition of claim 1, in the form of a mascara.

3. The composition of claim 1, in the form of a stick.

4. The composition of anyone of claims 1 to 3, further comprising at least one colorant

5. The composition of anyone of claims 1 to 4, wherein the at least one oil-soluble polar modified polymer is present in an amount of from 1 % to 30% of the total weight of the composition.

6. The composition of anyone of claims 1 to 5, wherein the at least one oil-soluble polar modified polymer is a wax, and preferably is a polypropylene and/or polyethylene-maleic anhydride modified wax.

7. The composition of anyone of claims 1 to 6, wherein the polyamine is present in an amount of from 0.05% to 20% by weight, based on the weight of the composition.

8. The composition anyone of claims 1 to 7, wherein the composition is made using from 0.01 to 5% by weight, based on the weight of the composition, of the polyamine,

9. The composition of anyone of claims 1 to 8, wherein the polyamine is a branched polyethyleneimine.

10. The composition of anyone of claims 1 to 9, wherein the aqueous polyurethane dispersion is present in an amount of from about 1% to about 35% of the total weight of the composition.

11. The composition of anyone of claims 1 to 10, wherein the at least one aqueous polyurethane dispersion comprises a hydrophilic portion.

12. The composition of anyone of claims 1 to 11, wherein the at least one aqueous polyurethane dispersion is selected from the group consisting of polyurethane-34, polyurethane-35 and polyurethane-32.

13. The composition of anyone of claims 1 to 12, further comprising water, and preferably in an amount of from 0.1 to 50% by weight, based on the weight of the composition, and more preferably in an amount of from about 5% to about 50% by weight, based on the weight of the composition.

14. The composition of claim 13, in the form of an emulsion.

15. The composition of anyone of claims 1 to 14, wherein the oil-soluble polar modified polymer and the polyamine form a reaction product.

16. A method of making up eyelashes comprising applying the composition of anyone of claims 1, 2 and 4 to 15 to eyelashes in an amount sufficient to makeup the eyelashes.

17. A method of making up lips or skin comprising applying the composition of anyone of claims 1, 3 to 15 to lips or skin in an amount sufficient to makeup the lips or skin.
